# EUROPEAN PATENT SPECIFICATION

(11) **EP 1 446 481 B1**
(45) Date of publication and mention of the grant of the patent: **21.05.2008**
(21) Application number: 02803152.4
(22) Date of filing: 07.10.2002
(51) Int. Cl.: C12N 11/08

(54) **MATERIALS AND METHODS FOR REDUCING OXALATE CONCENTRATIONS IN FLUIDS**
MATERIALIEN UND METHODEN ZUM REDUZIEREN DER KONZENTRATION VON OXALAT IN FLUIDEN
MATERIAUX ET PROCEDES PERMETTANT DE REDUIRE LES CONCENTRATIONS D'OXALATE DANS DES FLUIDES

(30) Priority: 05.10.2001 US 327544 P
(43) Date of publication of application: 18.08.2004
(73) Proprietor: OxThera, Inc.,, Alachua, FL 32615 (US)
(72) Inventor: SIDHU, Harmeet, Gainesville, FL 32606 (US)
(74) Representative: Johansen, Marianne
(86) International application number: PCT/US2002/032087
(87) International publication number: WO 2003/042380

(56) References cited:
- WO-A-98/07922
- DE-A- 3 030 185
- DE-A- 3 204 284
- US-B1- 6 177 478
- US-B1- 6 281 252
- POTEZNY N ET AL: "URINARY OXALATE DETERMINATION BY USE OF IMMOBILIZED OXALATE OXIDASE IN A CONTINUOUS-FLOW SYSTEM" CLINICAL CHEMISTRY, AMERICAN ASSOCIATION FOR CLINICAL CHEMISTRY. WINSTON, US, vol. 29, no. 1, 1983, pages 16-20, XP002044107 ISSN: 0009-9147
- CHANDRAN P ET AL: "Improved determination of urinary oxalate with alkylamine glass bound barley oxalate oxidase" BRAUWELT, NUERNBERG, DE, vol. 85, no. 1, 23 January 2001 (2001-01-23), pages 1-5, XP004315103 ISSN: 0168-1656
- PUNDIR C S ET AL: "Immobilization of sorghum leaf oxalate oxidase onto alkylamine and arylamine glass." CHINESE JOURNAL OF BIOTECHNOLOGY. UNITED STATES 1999, vol. 15, no. 2, 1999, pages 129-138, XP009007458 ISSN: 1042-749X

## Description

### Background of Invention

Oxalic acid (oxalate) is a natural by-product of metabolic processes in vertebrate animals and many consumable plants. Unfortunately, oxalate is not properly degraded in a significant portion of humans, a condition which may result in the formation of kidney stones in those persons. It is estimated that 70% of all kidney stones are composed of oxalate. Approximately 12 percent of the U.S. population will suffer from a kidney stone at some time in their lives. Persons suffering from, and at risk for, developing kidney stones, as well as patients with lipid malabsorption problems (e.g., sprue, pancreatic insufficiency, inflammatory intestinal disease, bowel resection, etc.), tend to have elevated levels of urinary oxalate.

During end-stage renal disease (ESRD), oxalate accumulates resulting in hyperoxalemia and secondary oxalosis (Tomson, C.R., et al., 1989, Clin Nephrol 32:87-95). To provide the functions of a normally functioning kidney, hemodialysis provides clearance of plasma oxalate and plasma calcium oxalate. However, hemodialysis treatments do not totally eliminate the oxalate and calcium oxalate (Marangella et al., 1992, Nephron. 60:74-80). Without hemodialysis, patients with ERSD and primary hyperoxeluria (PH) have a higher risk of progressive systemic oxalosis (Hoppe et al., 1999, Kidney International 56:268-274).

In addition to the formation of stones in the urinary tract, the presence of oxalate in fluids can be problematic in a number of situations. For example, it is well established that the formation of calcium oxalate deposits on stents and catheters can compromise the ability of these devices to perform their functions, and can be a contributing factor in the establishment of microbial infections. Similarly, the formation of oxalate deposits also contributes to reduced efficiency and spoilage in fermentation processes, including in the brewery industry.

Of particular significance with respect to one embodiment of the current invention is the deleterious affects of oxalate on indwelling catheters and stents. Catheters and stents are commonly used in medical procedures including, for example, for the management of urinary and peritoneal dialysis flow, haemodialysis, and drainage of renal calculi after laser or lithotripsy treatment. The use of long-term indwelling urinary catheters and stents in the urinary tract suffers limitations due to the occurrence of encrustation and bacterial adhesion which increases the risk of blockage of the device and urinary tract infections (UTI).

The pathogenesis of UTI associated with urinary devices often involves the formation of biofilms. An important step in the biofilm formation is the deposition of urinary components onto the device in the form of a conditioning film. (Fuse et al. 1994, J. Urol. 151:1703-1706; Tiezer et al. 1998, J. Urol. 160:876-881; Santin et al. 1999, Biomaterials, 20:1245-1251). Within hours, this film can consist of encrustation deposits that interfere with the urine flow. Biochemical and optical analyses of such encrustation has revealed the presence of calcium oxalate.

Epidemiological studies on the incidence of stent encrustation in various urological conditions have indicated urolithiasis as a major risk factor for stent encrustation. A significantly higher incidence of encrustation has been reported in stone formers (Keane et al. 1994, Br. J. Urol. 73:687-691; Robert et al. 1997, Urol. Int. 58:100-104). Keane et al. report encrustations on 58% of stents and the major risk factor for stent encrustation was a history of urolithiasis. This is apparently due to supersaturation of a stone former's urine with the lithogenic components mainly calcium and oxalate. The oxalate concentration of urine is the most important parameter for calcium oxalate saturation. Since there are >10 calcium ions for each oxalate ion in urine, small increases in oxalate result in exceeding the threshold of calcium oxalate supersaturation causing its crystallization.

As patients usually receive antibiotics prior to placement of indwelling medical devices the normal urethral flora has often been replaced with uropathogenic drug resistant organisms adept at colonizing surfaces. The presence of a catheter or a stent can also facilitate the ascent of the bacteria into the renal pelvis and tissues. Once device-related infections become established they are generally difficult to treat and usually necessitate the removal of the device.

In addition to being a site of infection, a large number of stents have been shown to have impassable blockage (El-faqih et al. 1991, J. Urol. 146:1487-1491). Encrustation deposition on synthetic materials in the urinary tract can occur both in infected and sterile urine. The mechanism of encrustation in infected urine is analogous to formation of urinary stones: urease producing organisms elevate the urinary pH by hydrolysis of urea thereby creating an alkaline environment where magnesium and calcium readily precipitate out of solution forming crystals.

In sterile urine, the formation of encrustation on the biomaterials appears to be dependent on both urinary constituents and the properties of the synthetic material (Ramsay JWA 1987, Br. J. Urol. 1987, 66:66-70; Denstedt et al. 1998, J. Endourol. 12:493-500). A wide variety of ureteral stents and urethral catheters are available. Silicone is a highly biocompatible material and is used for the manufacture of urethral catheters as well as ureteral stents. Although there has been research into the development of materials with modified surface properties, none of these has been established to be encrustation resistant (Tunney et al. 1996, Biomaterials, 17:1541-1547). Some of the commonly used devices are the standard silicone latex device (Sof Flex, Cook Urology, Indiana), hydrophilic stents (Boston Scientific, MA) and the low surface energy stents (LSe,Cook Urology, IN) that have been specially designed to lower the risk of encrustation. Extensive studies carried out in the Urology ESWL Center at University of Western Ontario have documented encrustation and biofilm processes on all the above commonly used biomaterials (Tiezer et al. 1998, J. Urol. 160:876-881; Wollin et al. 1998, J. Endourol. 12:101-111; Teizer et al. 1998, Biomater. 43:321-330; Reid et al. 1992, J. Urol. 148:1592-1594).

One of the more recent developments is the use of hydrogel coatings on indwelling medical devices. These hydrophilic polyurethane polymers swell on contact with water and retain a significant proportion of water within their polyanionic structure. This is believed to reduce friction and stent encrustation. Studies have also been undertaken with non-ionic synthesized hydrogels, such as polyacrylamide, polyvinyl alcohol, polyethylene glycol and polymethoxy-PEG methacrylate and with ionic hydrogels such as crosslinked polyacrylamide-dimethyl-aminoethyl methacrylate copolymers (Kulik E. and Y. Ikada, 1996, J. Biomed. Mater. Res. 30:295-304). A polyethylene glycol-based hydrogel linked to serum albumin has been reported to be a suitable matrix for enzyme immobilization on the biomedical devices (D'Urso EM et al. 1995, Immobil. Biotechnol 23:587-595).

Surface immobilization of enzymes is a convenient and cost-effective method for allowing simple recovery and reuse of an enzyme in industrial and laboratory settings. Enzymes have been covalently linked to substrates using a variety of bioconjugation techniques. Common substrates include organic materials such as polycarbonate and polysaccharide materials (including chitosan) and inorganic materials such as silica glass. These substrates are often surface functionalized in order to covalently link enzymes.

Silicone elastomer is a commonly used material in urinary catheter manufacturing because of its bioinertness, low coefficient of friction, and flexibility. Attempts have been made to modify the silicone elastomer surface in order to produce a more hydrophilic (Urban, M.W., M.T. Stewart, 1990, "ATR FT-IR studies of gas plasma modified silicone elastomer surfaces" J Appl Polym Sci. 39:265-283; Howard I.H. 1991, "Surface modification of polymers by radio frequency plasma, Ph.D. dissertation, University of Florida, page 76; and Lee, S.D., G.H. Hsiue, C.Y. Kao, 1996, Preparation and characterization of a homobifunctional silicone rubber membrane grafted with acrylic acid via plasma-induced graft copolymerization" J. Polym Sci Pol Chem 34:141-148), functionalized (Mutlu, M. et al., 1991, "Matrix surface modification by plasma polymerization for enzyme immobilization" J. Mater Chem 1:447-450; Gaboury, S.R., M.W. Urban, 1993, "Analysis of gas plasma-modified poly-(dimethylsiloxane) elastomer surfaces-attenuated-total-reflectance Fourier-transform infrared-spectroscopy" *Adv Chem* Ser777-790; and Rau, K.R., 2000, "Surface modification of biomaterials by pulsed laser ablation deposition and plasma/gamma polymerization" Ph.D. dissertation, University of Florida, Gainesville, FL), or graft polymerized (Lee, S.D., G.H. Hsiue, C.Y. Kao, 1996, "Preparation and characterization of a homobifunctional silicone rubber membrane grafted with acrylic acid via plasma-induced graft copolymerization" J. Polym Sci Pol Chem 34:141-148; Lee, S.D., G.H. Hsiue, C.C. Wang, 1994, "Characterization of plasma-induced graft-polymerization of 2-hydroxyethyl methacrylate onto silicone rubber" J. Appl Polym Sci 54:1279-1287; Lee, S.D. et al., 1996, "Plasma-induced grafted polymerization of acrylic acid and subsequent grafting of collagen onto polymer film as biomaterials" Biomaterials 17:1599-1608; Ksiue, G.H. et al., 1998 "Surface characterization and biological properties study of silicone rubber membrane grafted with phospholipid as biomaterial via plasma induced graft copolymerization" J Biomed Mater Res 42:134-147; and Langefeld, S. et al., 1999, Functionally adapted surfaces on a silicone keratoprosthesis" Int. J. Artif Organs 22:235-241) surface. Surface modification has been conducted in various ways including chemical reaction, UV irradiation, gamma irradiation, and radio-frequency plasma discharge (RFPD). RF plasma discharge is a useful technique for changing the surface properties of a material without modifying the bulk properties in order to enhance biocompatibility, such as changes in protein adsorption, or it can be used to increase desirable cell adhesion and growth to the biomaterial surfaces (Lee, S.D., G.H. Hsiue, C.C. Wang, 1994, "Characterization of plasma-induced graft-polymerization of 2-hydroxyethylmethacrylate onto silicone rubber" J. Appl Polym Sci 54:1279-1287; Ksiue, G.H. et al., 1998 "Surface characterization and biological properties study of silicone rubber membrane grafted with phospholipid as biomaterial via plasma induced graft copolymerization" J Biomed Mater Res 42:134-147; Langefeld, S. et al., 1999, Functionally adapted surfaces on a silicone keratoprosthesis" Int. J. Artif Organs 22:235-241; and Mason, M. et al., 2000, "Attachment of hyaluronic acid to polypropylene, polystyrene, and polytetrafluoroethylene" Biomaterials 21:31-36).

Many of the recent advances in the development of indwelling medical devices are focused on the prevention of bacterial infection. Coating or impregnating the biomaterials with antimicrobial substances such as heavy metals, silver oxide and antibiotics could provide reduction in the incidence of infection especially for patients requiring short term insertion of a device (Johnson JR et al. 1990, J. Infect. Dis. 162:1145-1150; Gilchrist T. et al. 1991, Biomaterials 12:76-78).

As noted above, the presence of oxalate in fluids can cause problems in environments other than indwelling medical devices. In the fermentation industry, scale is formed by the surface deposition on equipment of 1) water-insoluble sulfate and carbonate salts of calcium and magnesium (mineral scale) and 2) calcium oxalate that forms during the normal fermentation process. The presence of scale on equipment is not serious if addressed early. If left unchecked, however, it can result in significant operational problems.

As in the case of medical devices, scale represents a microbiological hazard. The crystalline structure of scale affords significant protection for spoilage organisms (such as Pediococcus and Lactobacillus) from the effects of cleaning and sterilizing agents. Second, scale formation significantly reduces heat transfer across equipment surfaces. This in turn reduces the efficiency of heating and cooling operations, which translates into increased turn-mund time, reduced production capacity, and inflated energy costs. Third, scale is especially detrimental to boilers, because it is rapidly deposited under the harsh conditions in which a boiler operates. This buildup can lead to boiler failure and possible explosion.

Unfortunately, the chemical reactions that lead to scale formation are inherent to the fermentation process. For example, scale is formed via a number of chemical reactions that occur throughout the brewing process. One Form of scale, commonly known as beer stone, is brought about by the reaction of calcium with oxalic acid under the conditions of mashing. Oxalic acid is an organic acid constituent of barley. In addition to scale formation, calcium oxalate has been linked to gushing and colloidal instability of packaged beer.

Today there are a number of options for dealing with scale in the brewery. These include ion exchange, sequestering agents, and acid cleaners. The subject invention provides an alternative method for removing oxalate from fluid systems.

### Brief Summary

The subject invention provides materials and use of at least one oxalate-degrading enzyme in the preparation of a composition for use in methods for reducing oxalate concentrations in biological fluids. In a preferred embodiment, oxalate-degrading enzymes are immobilized on a surface of a dialysis membrane or an in-dwelling medical device and provide an efficient means of lowering free oxalate in a fluid which comes in contact with the surface. In specific embodiments exemplified herein, oxalate-degrading enzymes are attached to surfaces which are contacted with biological fluids. These surfaces may be on, for example, catheters, stents, or dialysis membranes.

Oxalate-degrading enzymes may also be attached to surfaces which come into contact with fluids involved in a fermentation process. These modified surfaces can thus be used to reduce the incidence of scale.

As described herein, oxalate-degrading enzymes can be coated onto materials used for the manufacture of devices for an efficient *in situ* degradation of oxalate to prevent the initial steps of calcium oxalate precipitation leading to encrustation. This approach can be used to prevent the blockage of the devices and the risk of microbial infection.

In one embodiment of the subject invention, radio-frequency plasma discharge (RFPD) is used to activate and functionalize an inert silicone elastomer surface. The surface is then coated with 3-aminopropyltriethoxysilane (AMEO) to derivatize the elastomer surface with an amine functionality to provide a site for coupling oxalate oxidase amine groups.

### Detailed Disclosure

The subject invention provides materials and use of at least one oxalate-degrading enzyme in the preparation of a composition for use in methods for reducing the oxalate concentration in biological fluids. Notably, the present invention provides the use of at least one oxalate-degrading enzyme in the preparation of a composition for immobilizing on a surface, for contacting with a biological oxalate-containing fluid for reducing the concentration of oxalate therein, wherein the oxalate-degrading enzyme is immobilized on the surface of a dialysis membrane or an in-dwelling medical device. This reduction in oxalate concentration is achieved by degrading the oxalate, or otherwise removing oxalate from the fluid. In a preferred embodiment, oxalate is degraded in biological fluids. Specifically exemplified herein are stents and catheters, catheters which have been modified to reduce oxalate concentrations in surrounding fluids thereby reducing or eliminating encrustation.

A further aspect of the subject invention pertains to dialysis membranes which have been modified to degrade oxalate thereby reducing the oxalate concentration of the surrounding fluid and increasing the efficiency of the dialysis procedure.

Oxalate may also be degraded in other fluids including fermentation broths. Specifically exemplified in the context of fermentation broths is the degradation of oxalate from fluids involved in the brewery process.

In a preferred embodiment of the subject invention, surfaces which will come into contact with a fluid are modified by associating oxalate-degrading enzymes with these surfaces. The surfaces which are modified in accordance with the subject invention may be, for example, made of a polymeric material. In one embodiment, the surface comprises silicone rubber.

The methods of the subject invention are distinct from the attachment of enzymes to surfaces for the purpose of conducting assays. Thus, the methods of the subject invention provide functional advantages through the reduction of the oxalate concentration in a fluid. The functional advantage may be, for example, a reduction in: the formation of calcium oxalate deposits, microbial infections, encrustation, bacterial adhesion, and scale formation.

The association of the enzymes with the surface may be by, for example, direct attachment, attachment through a linker, or by incorporation within a coating on the surface. There are a variety of methods, known to those skilled in the art for associating the oxalate-degrading enzymes with a desired surface. The important requirement of the method of association is that the ability of the enzyme (or enzymes) to degrade oxalate must be retained. So, for example, methods are known to those skilled in the art for directly attaching enzymes to synthetic materials. Alternatively, the enzymes may be attached to the desired surface through a linker. A great number of such linkers are known to those skilled in the art and include dendrimers such as those described in U.S. Patent No. 6,080,404.

In a further embodiment, the oxalate-degrading compounds may be entrapped within a coating. The coating may be, for example, those described in U.S. Patent Nos. 5,554,147; 5,607,417; and 5,788,687. These patents also describe various agents which can be entrapped within a device coating and used in conjunction with the oxalate-degrading enzymes of the subject invention.

Although significant research is being done on biomaterial modifications of stents and catheters, none of the currently used devices reduces the levels of encrustation satisfactorily. Advantageously, the devices of the subject invention can be in patient populations undergoing endourological procedures. These devices are particularly advantageous for use by patients who are prone to oxalate stone formation and those suffering from advanced malignant obstruction.

Silicone rubber (SR), is used for many biomedical devices because it has excellent biocompatibility, provides flexibility and long-term mechanical stability within the physiological environments. To prevent rapid deposition of a conditioning film in the urinary environment, hydrogel coatings have been applied to SR devices. Several techniques have been developed for the surface modification of SR via covalent grafting of various polymers (Lee, S et al. 1996, Journal of Polymer Science: Part A: Polymer Chemistry 34:141-148; De Fife KM et al. 1999, J. Biomed. Material. Res. 44:298-307; DiTizio, V et al. 1998, Biomatenials, 19:1877-1884; Langefeld, S et al. 1999, Int. J. Art. Organs, 22: 235-241). The surface of SR has been activated to provide functional groups capable of linking to polymerizable monomers. Activation can be accomplished using conventional energy sources, such as cobalt-60, radiofrequency or microwave gas discharge; and plasma discharge (De Fife KM et al. 1999, J. Biomed. Material. Res. 44: 298-307). Additionally, photochemical reactions using UV and redox reagents have been used to chemically modify an SR surface (DiTizio, V et al. 1998, Biomaterials 19:1877-1884).

With argon-plasma treatment, radicals are produced on the silicone surface, and then functional groups are produced by exposing the sample to gases, such as oxygen to produce peroxides, and ammonia to produce amide groups. Langefeld *et al* (Langefeld, S et al. 1999, Int. J. Art. Organs 22:235-241) have used the plasma treatment technique to produce surface hydroperoxides to initiate graft polymerization of polyacrylic acid (PAA) and polyglycidylmethacrylate, and have developed a method to covalently immobilize fibronectin to activated PAA. This method is used as a means of covalently immobilizing the oxalate degrading enzymes directly to the SR and to a hydrogel matrix of PAA.

SR can also be modified with a polyethylene glycol (PEG)-gelatin hydrogel. In one embodiment, this coating can be used to entrap an antibiotic. Examples of antibiotics which can be used include flouroquinolones, β-lactams, and cephalosporins. The antibiotics may be entrapped within liposomes. A combination of this antimicrobial coating along with the oxalate-degrading enzymes can be used to address two types of catheter encrustation, that is, bacterial-induced deposits of struvite and calcium phosphate, and calcium oxalate deposits from a non-infected environment.

In one embodiment of the subject invention, oxalate-degrading enzymes can be covalently bound to a radio-frequency plasma surface-modified silicone elastomer. These surface-modified materials can be used, for example, in the prevention of calcium oxalate encrustation on urological biomaterials. The oxalate-degrading enzyme only needs to affect the microenvironment surrounding the surface of the catheter in order to prevent the formation of calcium oxalate crystals at the surface. In addition, the production of H₂O₂, through OXO-mediated free oxalate degradation, may provide an antimicrobial affect in the urinary environment.

### Oxalate Degrading Enzymes

There are three main classes of oxalate degrading enzymes. Oxalate oxidase, is expressed in higher plants and it catalyzes the oxygen dependent oxidation of oxalate to CO₂ with concomitant formation of H₂O₂. A rapid three step purification procedure has been developed to obtain oxalate oxidase from barley roots (Kotsira VP and YD Klonis, 1997, Arch. Biochem. Biophys. 340:239-249). The gene encoding the barley root oxalate oxidase has been cloned, sequenced and expressed (Thompson C et al. 1995, Euphytica 85:169-172).

Oxalate decarboxylase, the second class of oxalate metabolizing enzymes, is mainly present in fungi (Shimazono H., 1955, J. Biochem. 42:321-340). Fungal oxalate decarboxylase catalyzes the degradation of free oxalate to CO₂ and formate. This enzyme has been reported in several fungi, including *Myrothecium verrucaria,* certain strains of *Aspergillus niger,* and white rot fungus, *Coriolus versicolor.* The gene encoding the *Flammulina velutipes* oxalate decorboxylase has been cloned and sequenced (Mehta A and A. Datta, 1991, J. Biol. Chem. 266:23,548-53; International patent no. WO 98/42827).

The bacterial enzyme for oxalate degradation, oxalyl-CoA decarboxylase, is active on the CoA-activated substrate and converts it into formyl-CoA. A formyl-CoA transferase then acts to exchange formate and oxalate on CoA. These enzymes have been studied in the oxalate degrading bacteria, *Pseudomonas oxalaticus* present in the soil (Qyayle PR et al. 1961, Biochemical J. 78:611-615) and in *Oxalobacter formigenes,* residing in the gastrointestinal tract of vertebrates, including humans (Allison, M.J. et al. 1985, Arch. Microbiol. 141:47). *O. formigenes* has been shown to play a symbiotic relationship with its hosts by regulating oxalic acid absorption in the intestine as well as oxalic acid levels in plasma. As a result the absence of this bacteria has been found to be a risk factor in oxalate related disorders like recurrent idiopathic calcium oxalate urolithiasis (Sidhu, H. et al., 1999 J. Am. Soc. Neph. 10:S334-S340; Kleinschmidt, K. et al., 1993, In: Urolithiasis 2, Plem Press, NY.) and enteric hyperoxaluria secondary to jejuno-ileal bypass surgery, cystic fibrosis and inflammatory bowel disease (Allison, M.J. et al. 1986, J. Nutr. 116:455-460; Sidhu, H. et al. 1998, Lancet 352:1026-1029). This bacterium is highly specific in utilizing only oxalate as its source of energy for its survival and growth. As a result the oxalate degrading enzymes oxalyl-CoA decarboxylase and formyl-CoA transferase comprise about 20 to 30% of its cellular proteins. Both the proteins as well as the membrane transporter for the oxalate-formate exchange have been purified and well characterized (Baetz AL and MJ Allison, 1989, J. Bact. 171:2605-2608; Baetz AL and MJ Allison, 1990, J. Bact. 172:3537-3540; Ruan ZS et al, 1992, J. Biol. Chem. 267:10537-19543). Also, the genes for all three proteins have been cloned, sequenced and expressed as biologically active recombinant proteins (Abe, K. et al., 1996, J. Biol. Chem. 271: 6789-6793; Lung H.Y. et al., 1994, J. Bact. 179:3378-3381; Sidhu, H. et al., 1997, J. Bact. 179:3378-3381).

Patents describing various oxalate-degrading enzymes and the genes encoding these enzymes include U.S. Patent Nos. 5,912,125; 6,090,628; and 6,214,980.

### MATERIALS AND METHODS

### Silicone Elastomer

Silicone elastomer (MDX4-4210, medical grade elastomer, Dow Coming, Midland, MI, supplied by Factor II, Inc., Lakeside, AZ) was prepared following the manufacturer's instructions. Briefly, the elastomer was cast into 2-mm-thick sheets by curing the resin between acrylic plates separated by a 2-mm spacer. The prepared sheets were allowed to cure for 48 h at room temperature. Discs of 10-mm diameter were cutfrom the cured sheets with the use of a cork boring tool (Boekel, Feasterville, PA). The discs were extracted for 48 h in HPLC grade hexanes (Fisher Scientific Co., Pittsburgh,PA) to remove unreacted species.

### Surface Modification

The radio frequency (RF) plasma discharge system consisted of a bell-jar-type reaction chamber, a sample stand, a vapor inlet port, a vacuum system with liquid nitrogen cold-trap, an RF power generator (RF Plasma Products, Inc., model HFS 401 S) operating at a fixed frequency of 13.56 MHz with a maximum output of 500 W, and a matching network to coordinate the impedance of the plasma discharge with the RF power generator. Silicone elastomer discs were placed in the chamber, which was subsequently brought to a vacuum of 40 mtorr andpurged five times with Ar gas for 15 s at 1000 standard cm³/min. The final Ar gas purge was ignited to form plasma to clean and activate the silicone discs for 15 min at 50 mtorr, 50 watts power. Ultra pure water vapor was slowly added to the chamber with the use of a needle valve to replace the Ar gas in the plasma (15 min at 50 mtorr, 50 watts power). Following plasma treatment, discs were rinsed in 100% ethanol for 15 min, then reacted with a 2% (v/v) solution of 3-aminopropyltriethoxysilane (AMEO; Sigma Chemical Co., St. Louis, MO) in 95% ethanol for 45 min, and then rinsed with 100% ethanol. The discs were left to cure 16-24 h in ambient conditions.

### Surface Characterization

The surface of the plasma treated discs was characterized with the use of underwater captive air contact angle goniometry and x-ray photoelectron spectroscopy (XPS). Contact angle measurements were made in ultra pure water with the use of a Rame'-Hart A-100 goniometer. Three air bubbles of approximately similar size were introduced from below onto the silicone surface with the use of a bent micro syringe. The contact angle of each was measured immediately as the angle made between the air bubble and the surface of the modified silicone disc. A total of 21 measurements were made from 7 discs from the same plasma treatment, from which the average contact angle was determined. The change in surface elemental chemistry of unmodified, plasma-treated, and plasma-treated-AMEO-coated silicone elastomer was determined with the use of XPS. A low resolution survey scan and a high-resolution elemental scan was performed for each sample with the use of a Kratos Analytical XSAM 800 with a DS800 data-acquisition system. The Kratos was equipped with a Mg K_{α} X-ray source operating at a pass energy of 1253.6 eV, with the use of 15 kV and 9-mA current. Spectra were obtained at a take-off angle of 90° relative to the sample surface. Elemental analysis, providing relative atomic concentrations, was performed from the relative peak areas of the carbon (C1s), oxygen (O1s), silicon (Si2p), and nitrogen (N1s) peaks. The carbon peak was used to calibrate peaks for high-resolution scans.

### Enzyme Immobilization

The oxalate-degrading enzyme, oxalate oxidase (OXO), from barley seedlings (Sigma Chemical Co., St. Louis, MO), was used. The enzyme was covalently bound to the activated silicone elastomer discs with the use of 2.5% (v/v) glutaraldehyde (Sigma Chemical Co., St. Louis, MO) in 0.01-M phosphate-buffered saline (PBS), pH 7.4. Plasma treated discs were placed into separate wells of a 24-well tissue culture plate. The discs were washed twice (5 min each) with PBS on a rocker bed under slight agitation. The 2.5% glutaraldehyde solution was added to each disc in a well, and the plate was incubated for 1 h at room temperature, under slight agitation. The discs were subsequently washed three times with PBS (5 min each), followed by two washes (5 min each) with 45-mM sodium succinate buffer at pH 4.0. The discs were then transferred to a clean tissue culture plate. To each disc, 1 ml of a 100-µg/ml oxalate oxidase solution, prepared in 45-mM sodium succinate buffer, pH 4.0, was added. The same amount of enzyme was added to a well with no disc, which served as a control reaction for enzyme activity analysis. The tissue culture plate was incubated on a rocker bed at 4 °C for 48 h at 20 rpm. Following incubation, the enzyme solution was aspirated and the discs were washed with sodium succinate buffer (5 min). The discs were tested for estimation of bound protein and enzymatic activity.

### Immobilized Enzyme Characterization

The OXO activity assay was determined by the method of Requena and Bornemann (Requena, L. et al., 1999, Biochem J 343:185-190). The assay is based on the colorimetric determination of H₂O₂ produced during degradation of oxalate by OXO. Discs with immobilized enzyme and 10 µg of the free incubated enzyme, in individual wells of a clean 24-well tissue culture plate, were incubated with 1 ml of 40 mM potassium oxalate in sodium succinate buffer, pH 4.0 at 37 °C (30 min) at 100 rpm. The samples were removed and boiled to quench the enzymatic reaction, and allowed to cool to room temperature. A volume of 0.5 ml of the above reaction mixture was added to a 1-ml disposable cuvette containing 0.5 ml 10-mM ABTS (2-2'azinobis-3-ethylbenzthiazoline- 6-sulphonic acid) and 10 µl 2400-U/ml horseradish peroxidase, both in 45-mM sodium succinate buffer, pH 4. The cuvettes were covered and incubated at room temperature for 15 min. The absorbance was determined at 650 nm, with the use of a Shimadzu UV160 spectrophotometer, against a blank of buffered potassium oxalate, ABTS and horseradish peroxidase. A molar extinction coefficient of 10,000 M⁻¹cm⁻¹ was used to calculate the activity of oxalate oxidase. One unit of activity was defined as the amount of enzyme required to degrade 1 µmole of oxalate per minute.

The amount of enzyme covalently immobilized onto the silicone elastomer surface was determined with the use of the QuantiPro^{™} BCA (bicinchoninic acid) assay kit (Sigma Chemical Co., St. Louis, MO). Silicone elastomer discs coated with OXO were incubated with 1 ml QP BCA working reagent tissue culture plate wells at 37 °C (2 h) then allowed to cool to room temperature, and the absorbance was read at 562 nm. The amount of immobilized enzyme was extrapolated from a standard curve of BSA solutions.

### Modified Robbins Device

The ability of OXO-coated silicone elastomer to prevent calcium oxalate encrustation, was evaluated in a simulated urinary environment with the use of a continuous-flow encrustation model, a modified Robbins device (MRD). The device, consists of a 23-cm acrylic block, enclosing a 1.0 X 1.5-cm channel, with tubing connectors at each end. A 0.5-cm-thick acrylic sheet, with 10 predrilled 1-cm-diameter holes, is secured to the acrylic block with screws; to prevent leaking, a rubber gasket is placed between the two acrylic sections. The discs are placed in plugs, each with a 2-mm-deep well, which are inserted into the 1-cm holes, allowing the inserted discs to remain flush with the acrylic top section, maintaining laminar flow within the device. Artificial urine (AU), the components of which are presented in Table I, was pumped through the modified Robbins device at a rate of 0.8 ml/min. Two solutions of AU were prepared, one containing sodium oxalate and the other containing calcium chloride, to prevent calcium oxalate crystallization. The two solutions were mixed as the artificial urine was pumped to the MRD, resulting in a mixed solution with a pH of 6.0 and a final relative supersaturation of 8 as calculated by EQUIL (Werness, P.G. et al., 1985, J. Urol 134:1242-1244).

Four OXO-coated discs and four control (no enzyme) discs were fitted into the sample plugs and incubated with AU for 6 days. One enzyme-coated and one control disc were removed at Days 2, 4, and 6 and rinsed with 45-mM sodium succinate buffer, pH 4.0. These discs were tested for enzymatic activity as previously mentioned and then tested for degree of encrustation. At Day 6 the remaining discs were rinsed with distilled water, allowed to dry, and prepared for scanning-electron microscopy (SEM) and energy-dispersive X-ray spectroscopy (EDS).

| Table 1. Artificial Urine Composition, at pH6 and Final Relative Supersaturation of 8 | | | |
|---|---|---|---|
| Substance | Final Conc. (mmol/l) | Ion | Final Conc. (mmol/l) |
| NaCl | 105.50 | Na | 153.86 |
| KCl | 63.70 | K | 63.7 |
| Na₂SO₄ | 16.95 | Cl | 177.2 |
| NaH₂PO₄·H₂O | 3.23 | SO₄ | 20.8 |
| Na₃C₆H₅O₇·2H₂O | 3.21 | PO₄ | 3.23 |
| NaN₃ | 1.00 | Ca | 3.5 |
| MgSO₄ | 3.85 | Ox | 0.3 |
| Na₂C₂O₄ | 0.30 | Citrate | 3.21 |
| CaCl₂ | 4.00 | Mg | 3.85 |

### Encrustation Assessment

The discs were rinsed with deionized water and treated with 1 ml of 0.05 M HCl at 4 °C overnight (15-20 h) to dissolve the encrusted material. The HCl solutions with dissolved oxalate were tested for oxalate concentration using the Sigma Diagnostics® Kit ®for Oxalate (Sigma Chemical Co., St. Louis, MO). The oxalate concentration was used to quantitate the degree of encrustation at the disc surface. Structural and elemental analyses of disc encrustation were also performed with the use of scanning-electron microscopy (SEM) and energy-dispersive X-ray spectroscopy (EDS). Silicone elastomer samples were mounted on a 5/8- in. aluminum stub with a piece of conductive tape. The samples and stub were coated with gold/palladium with the use of standard conditions. Samples were analyzed with the use of a JEOL 6400 microscope operating at 15-kV accelerating voltage, an aperture setting of 2-3 and a condensor lens current range of 9-10 nA. Digital image acquisition was performed with the use of the accompanying Oxford microanalysis hardware with a Link ISISTM software package. EDS was performed for compositional analysis of the surface encrustation using the same hardware and software associated with the JEOL 6400 SEM.

Following are examples which illustrate procedures for practicing the invention. These examples should not be construed as limiting. All percentages are by weight and all solvent mixture proportions are by volume unless otherwise noted.

### Example 1 - Adsorption of enzyme to SR.

The oxalate-degrading enzymes used according to the subject invention can be adsorbed to a SR surface by immersing the substrate into a solution containing protein for several hours, at 37°C, with gentle stirring. Although protein adsorption onto SR is likely, one cannot expect to get high concentrations of the protein by this method. Ruggieri et al (81), have used an ionic surfactant, tridodecy-methyl-ammonium chloride (TDMAC), to complex heparin to hydrophobic catheter surfaces (latex, PVC, and PTFE). Although this method was successful for reducing bacterial adherence to the catheter surface, the heparin was found to leach off after about a week. Because of these inherent difficulties with these simple methods for enzyme immobilization, other, more permanent, methods of immobilizing the enzyme can be used. Alternative methods include:
1) SR can be activated within a plasma chamber and treated with ultrapure ammonia gas. The activated SR can then be dipped into a solution containing a coupling agent, such as glutaraldehyde or tris(hydroxymethyl)phosphine (THP). It has been shown that THP is a highly effective coupling agent for amine functionality, and it is less prone to hydrolysis than glutaraldehyde (Oswald, P.R. et al., 1998, Enzyme and Microbial Technology, 23:14-19). Finally, the modified silicone can be dipped into a solution containing the enzyme to link it to the coupling agent covalently through its amine basic residues. (Amino acid analysis shows that the oxalate degrading enzymes contain numerous such amine residues). Amine-containing side chains, such as lysine, arginine, and histidine, are typically exposed on the surface of proteins and can usually be derivatized with ease.
2) The methodology described by DiTizio et al (DiTizio, V. et al., 1998, Biomaterials, 19:1977-1884) can also be used. This method consists of applying a PEG-gelatin-liposome mixture to a silicone surface that has been pretreated with a thin layer of 4-azido-2,3,5,6-tetrafluorobenzoic (AFB) acid-modified gelatin. The AFB-gelatin can be synthesized, as outlined in their publication. The AFB-gelatin is immobilized on the silicone surface with UV light irradiation, and then the hydrogel mixture is applied by immersion of the coated biomaterial in an alkaline solution and crosslinked via reaction of the NPC-PEG (polyoxyethylene bis p-nitrophenyl carbonate) with the amine groups of the gelatin. This method can be adopted by replacing the liposome component with an oxalate degrading enzyme. The enzymes have amine groups which bind covalently with the NPC-PEG, along with the gelatin amine groups.
3) Polyacrylic acid (PAA) can also be grafted onto SR using argon-plasma technique (Langefeld, S. et al., 1999, Int. J. Art. Organs, 22:235:241). The enzyme can be covalently linked to the PAA by activation of the ends of grafted PAA chains with N(3-dimethylaminopropyl)-N'-ethylcarbodiimide hydrochloride.

### Example 2 - Immobilization of oxalate oxidase on silicone eastomer.

- A silicone elastomer surface was modified with RF plasma under Ar gas and then water vapor. Ar plasma resulted in a significant increase in surface hydrophilicity and relative oxygen content, as determined by contact angle and XPS analysis. Water-vapor plasma resulted in a further increase in the surface hydrophilicity and oxygen content, as compared to the Ar plasma treatment. Application of an AMEO coating to plasma treated silicone elastomer resulted in a relative decrease in surface hydrophilicity. Increased nitrogen content of the AMEO coated surface, as measured by XPS, indicated surface amination.

The active oxalate oxidase enzyme was immobilized on the aminated surface via glutaraldehyde bioconjugation. The immobilized OXO retained much of its native activity.

The effectiveness of the immobilized oxalate oxidase on PDMS discs toward prevention of calcium oxalate encrustation in the urinary environment was measured with the use of a modified Robbins device. Coating of the silicone discs with OXO led to 54% and 56% reduction in the concentration of oxalate on the encrusted material compared to the uncoated discs after 4 and 6 days of incubation in circulating artificial urine, respectively. The encrustation inhibition on the enzyme-coated surfaces was further confirmed by morphological and elemental analysis of the discs surfaces by SEM and EDS.

### Surface Modification

The captive air contact angle measurements and surface elemental compositions, as determined by XPS, are presented in Table II for the different plasma treatments.

| Table II. Captive Air Contact Angle Measurements and XPS Elemental Surface Analysis of Plasma-Treated Silicone Elastomer | | | | | |
|---|---|---|---|---|---|
| Plasma Treatment | Contact Angle (°) (mean ± S.D.) *n* = 21) | Atomic Concentration (%) | | | |
| | | O | C | Si | N |
| Control (none) | 86 ± 4 | 27.9 | 46.5 | 25.6 | n/d |
| Ar | 35 ± 7 | 35.7 | 37.2 | 27.0 | n/d |
| H₂O | 23 ± 5 | 37.9 | 36.5 | 25.5 | n/d |
| AMEO coated | 96 ± 9 | 32.0 | 39.0 | 26.3 | 2.8 |
| n/d = not detectable (< 0.5%) | | | | | |

Both Ar and H₂O plasma treatment produced surfaces with increased oxygen content and a corresponding decreased carbon content, as compared to control silicone elastomer, correlating with increasing hydrophilicity, as indicated by the decreased contact angle. H₂O plasma treatment, followed by AMEO coating, resulted in a strongly hydrophobic surface (contact angle of 96°), with oxygen and carbon amounts comparable to that of the control PDMS sample. The AMEO-coated surface was the only treatment that resulted in surface amination. The silicone content remained essentially unchanged after plasma treatment.

### Enzyme Immobilization

The amount of immobilized protein and its enzymatic activity, determined by the QP BCA protein estimation method, is presented in Table III.

| Table III. Enzymatic Activity of Oxalate Oxidase Immobilized on Silicone Elastomer | | | |
|---|---|---|---|
| | Total Protein (µg) | Activity (µmoles/min) | Specific Activity (U/mg protein) |
| Enzyme Sample | (mean ± S.D.) *(n* = 6) | (mean ± S.D.) (*n* = 6) | (mean ± S.D.) (*n* = 6) |
| Immobilized OXO | 20.8 ± 3.6 | 0.0004 ± 0.0001 | 0.019 ± 0.003 |
| Free OXO^{a} | 10.0 ± 0.0 | 0.0004 ± 0.0002 | 0.040 ± 0.016 |
| ^{a}Oxalate oxidase in solution incubated at 4°C for 48 h as a control. | | | |

On the average, 20.8 µg of oxalate oxidase (OXO) could be immobilized on an aminated 10mm diameter silicone disc via glutaraldehyde bioconjugation, which corresponds to about 0.26 µg/mm2. The immobilized protein was found to be enzymatically active and retained 47.5% of its native activity, as determined with the use of the free enzyme (Table III).

### Encrustation Assessment

Table IV shows the amount of resolubilized oxalate, representing the degree of encrustation, from the silicone elastomer discs incubated in the MRD for 6 days. After 2 days of incubation in the MRD, there was very little encrustation deposition on either control or OXO-coated discs. After only 4 days, the OXO-coated silicone elastomer discs significantly inhibited encrustation deposition, with a more pronounced effect at 6 days.

| Table IV. Quantitative Assessment of Degree of Encrustation on Silicone Elastomer Discs Incubated in Artificial Urine *in vitro* Using the Modified Robbins Device | | | |
|---|---|---|---|
| Sample | Time (days) | Degree of Encrustation^{a} | Percent change vs control (%) |
| | 2 | n/d | - |
| Control | 4 | 0.068 | - |
| | 6 | 0.115 | - |
| | 2 | n/d | n/d |
| OXO | 4 | 0.033 | 52 |
| | 6 | 0.053 | 54 |
| n/d = not detectable | | | |
| ^{a}Measured as oxalate concentration in encrusted material dissolved in 0.05-M HCl. | | | |

Scanning-electron microscopy (SEM) of the control disc PDMS surface showed some encrustation deposits, whereas SEM of the corresponding OXO-bound PDMS discs revealed fewer encrustation deposits. The encrustation deposits were predominantly shown to morphologically resemble calcium oxalate monohydrate crystals. Electron-dispersive spectroscopy (EDS) of both surfaces confirmed that the encrustation deposits contained calcium. The compositional mapping for calcium confirmed that there was more encrustation deposition on control PDMS as compared to OXO-bound PDMS samples.

## Claims

1. The use of at least one oxalate-degrading enzyme immobilizing on the surface of a dialysis membrane or an in-dwelling medical device to reduce the concentration of oxalate in a biological fluid.

2. The use of claim 1, wherein the biological fluid is blood or urine.

3. The use of claim 1 or 2 wherein the in-dwelling medical device comprises a catheter, a stent, or a dialysis membrane.

4. The use of any of claims 1 to 3, wherein the surface upon which the enzyme is immobilized is a polymeric material.

5. The use of any of claims 1 to 4, wherein the surface upon which the enzyme is immobilized is a silicone elastomer.

6. The use of any preceding claim, wherein the oxalate-degrading enzyme is attached to the surface through a linker.

7. The use of any preceding claim, wherein the surface further comprises an antimicrobial compound.

8. The use of any preceding claim, wherein the enzyme is oxalate oxidase, oxalate decarboxylase, oxalyl-CoA decarboxylase, or formyl-CoA transferase.

9. The use of any preceding claim, wherein multiple oxalate-degrading enzymes are immobilized on the surface.

10. The use of any of claims 3-9, wherein the device in-dwells in a mammal.

11. The use of claim 10, wherein the mammal is a human.

12. A surface to which at least one oxalate-degrading enzyme is attached and wherein said surface is on a dialysis membrane or an in-dwelling medical device.

13. The surface of claim 12, wherein the in-dwelling medical device comprises a stent or catheter.

14. The surface of claim 12 or claim 13, which is a polymeric material.

15. The surface of any of claims 12 to 14, wherein the oxalate-degrading enzyme is attached to the surface through a linker.

16. The surface of any of claims 12 to 15, wherein the surface further comprises an antimicrobial compound.

17. The surface of any of claims 12 to 16, wherein the enzyme is oxalate, oxidase, oxalate decarboxylase, oxalyl-CoA decarboxylase, or formyl-CoA transferase,

18. The surface of any of claims 12 to 17, wherein multiple oxalate-degrading enzymes are immobilized on the surface.

19. A medical device comprising a dialysis membrane, a stent or a catheter, the device comprising at least one surface to which at least one oxalate degrading enzyme is attached.

20. The medical device of claim 19, wherein the surface is a polymeric material.

21. The medical device of claim 19 or claim 20, wherein the oxalate-degrading enzyme is attached to the surface through a linker.

22. The medical device of any of claims 19 to 21, wherein the surface further comprises an antimicrobial compound.

23. The medical device of any of claims 19 to 22, wherein the enzyme is oxalate oxidase, oxalate decarboxylase, oxalyl-CoA decarboxylase, or formyl-CoA transferase.

24. The medical device of any of claims 19 to 23, wherein multiple oxalate-degrading enzymes are immobilized on the surface.

## Patentansprüche

1. Verwendung wenigstens eines oxalatabbauenden Enzyms, das auf der Oberfläche einer Dialysemembran oder einer permanenten (in-dwelling) medizinischen Vorrichtung immobilisiert ist, zum Reduzieren der Konzentration von Oxalat in einer biologischen Flüssigkeit.

2. Verwendung gemäß Anspruch 1, wobei es sich bei der biologischen Flüssigkeit um Blut oder Urin handelt.

3. Verwendung gemäß Anspruch 1 oder 2, wobei die permanente medizinische Vorrichtung einen Katheter, einen Stent oder eine Dialysemembran umfasst.

4. Verwendung gemäß einem der Ansprüche 1 bis 3, wobei die Oberfläche, auf der das Enzym immobilisiert ist, ein polymeres Material ist.

5. Verwendung gemäß einem der Ansprüche 1 bis 4, wobei die Oberfläche, auf der das Enzym immobilisiert ist, ein Silikon-Elastomer ist.

6. Verwendung gemäß einem der vorstehenden Ansprüche, wobei das oxalatabbauende Enzym über einen Linker an die Oberfläche gebunden ist.

7. Verwendung gemäß einem der vorstehenden Ansprüche, wobei die Oberfläche weiterhin eine antimikrobielle Verbindung umfasst.

8. Verwendung gemäß einem der vorstehenden Ansprüche, wobei es sich bei dem Enzym um Oxalat-Oxidase, Oxalat-Decarboxylase, Oxalyl-CoA-Decarboxylase oder Formyl-CoA-Transferase handelt.

9. Verwendung gemäß einem der vorstehenden Ansprüche, wobei mehrere oxalatabbauende Enzyme auf der Oberfläche immobilisiert sind.

10. Verwendung gemäß einem der Ansprüche 3 bis 9, wobei die Vorrichtung permanent in einem Säuger angebracht ist.

11. Verwendung gemäß Anspruch 10, wobei der Säuger ein Mensch ist.

12. Oberfläche, an die wenigstens ein oxalatabbauendes Enzym gebunden ist, wobei sich die Oberfläche auf einer Dialysemembran oder einer permanenten medizinischen Vorrichtung befindet.

13. Oberfläche gemäß Anspruch 12, wobei die permanente medizinische Vorrichtung einen Stent oder Katheter umfasst.

14. Oberfläche gemäß Anspruch 12 oder 13, bei der es sich um ein polymeres Material handelt.

15. Oberfläche gemäß einem der Ansprüche 12 bis 14, wobei das oxalatabbauende Enzym über einen Linker an die Oberfläche gebunden ist.

16. Oberfläche gemäß einem der Ansprüche 12 bis 15, wobei die Oberfläche weiterhin eine antimikrobielle Verbindung umfasst.

17. Oberfläche gemäß einem der Ansprüche 12 bis 16, wobei es sich bei dem Enzym um Oxalat-Oxidase, Oxalat-Decarboxylase, Oxalyl-CoA-Decarboxylase oder Formyl-CoA-Transrerase handelt.

18. Oberfläche gemäß einem der Ansprüche 12 bis 17, wobei mehrere oxalatabbauende Enzyme auf der Oberfläche immobilisiert sind.

19. Medizinische Vorrichtung, die eine Dialysemembran, einen Stent oder einen Katheter umfasst, wobei die Vorrichtung wenigstens eine Oberfläche umfasst, an die wenigstens ein oxalatabbauendes Enzym gebunden ist.

20. Medizinische Vorrichtung gemäß Anspruch 19, wobei die Oberfläche ein polymeres Material ist.

21. Medizinische Vorrichtung gemäß Anspruch 19 oder 20, wobei das oxalatabbauende Enzym über einen Linker an die Oberfläche gebunden ist.

22. Medizinische Vorrichtung gemäß einem der Ansprüche 19 bis 21, wobei die Oberfläche weiterhin eine antimikrobielle Verbindung umfasst.

23. Medizinische Vorrichtung gemäß einem der Ansprüche 19 bis 22, wobei es sich bei dem Enzym um Oxalat-Oxidase, Oxalat-Decarboxylase, Oxalyl-CoA-Decarboxylase oder Formyl-CoA-Transferase handelt.

24. Medizinische Vorrichtung gemäß einem der Ansprüche 19 bis 23, wobei mehrere oxalatabbauende Enzyme auf der Oberfläche immobilisiert sind,

## Revendications

1. L'utilisation d'au moins une enzyme de dégradation d'oxalate en vue de son immobilisation sur la surface d'une membrane de dialyse ou d'un dispositif médical implantable pour réduire la concentration en oxalate d'un fluide biologique.

2. L'utilisation selon la revendication 1, dans laquelle le fluide biologique est du sang ou de l'urine.

3. L'utilisation selon la revendication 1 ou 2, dans laquelle le dispositif médical implantable comprend un cathéter, une endoprothèse (stent) ou une membrane de dialyse

4. L'utilisation selon une quelconque des revendications 1 à 3, dans laquelle la surface sur laquelle l'enzyme est immobilisée est un matériau polymère.

5. L'utilisation selon une quelconque des revendications 1 à 4, dans laquelle la surface sur laquelle l'enzyme est immobilisée est un silicone élastomère.

6. L'utilisation selon une quelconque des revendications précédentes, dans laquelle l'enzyme de dégradation de l'oxalate est fixée à la surface par un agent de liaison

7. L'utilisation selon une quelconque des revendications précédentes, dans laquelle la surface comprend en outre un composé antimicrobien

8. L'utilisation selon une quelconque des revendications précédentes, dans laquelle l'enzyme consiste en oxalate oxidase. oxalate décarboxylase. oxalyl-CoA décarboxylase ou formyl-CoA transferase.

9. L'utilisation selon une quelconque des revendications précédentes, dans laquelle multiple enzymes de dégradation de l'oxalate sont immobilisées sur ladite surface

10. L'utilisation selon une quelconque des revendications 3 à 9, dans laquelle le dispositif est implanté chez un mammifère.

11. L'utilisation selon la revendication 10, dans laquelle le mammifère est un homme.

12. Une surface sur laquelle au moins une enzyme de dégradation d'oxalate est fixée et dans laquelle ladite surface est celle d'une membrane de dialyse ou d'un dispositif médical implantable

13. La surface selon la revendication 12 dans laquelle l'dispositif médical implantable consiste en un stent ou cathéter.

14. La surface selon la revendication 12 ou la revendication 13, formée d'un matériau polymère.

15. La surface selon une quelconque des revendications 12 à 14, dans laquelle l'enzyme de dégradation d'oxalate est fixée à la surface par l'intermédiaire d'un agent de liaison

16. La surface selon une quelconque des revendications 12 à 15, dans laquelle la surface comprend en outre un composé antimicrobien.

17. La surface selon une quelconque des revendications 12 à 14 ; dans laquelle l'enzyme consiste en oxalate, oxidase; oxalate décarboxylase, oxalyl-CoA décarboxylase, ou formyl-CoA transférase.

18. La surface selon une quelconque des revendications 12 à 17, dans laquelle les multiples enzymes de dégradation d'oxalates sont immobilisées sur ladite surface.

19. Un dispositif médical comprenant une membrane de dialyse, une endoprothèse (stent) ou un cathéter, le dispositif comprenant au moins une surface à laquelle au moins une enzyme de dégradation d'oxalate est fixée.

20. Le dispositif médical selon la revendication 19, dans laquelle la surface est formée d'un matériau polymère.

21. Le dispositif médical selon la revendication 19 ou selon la revendication 20, dans laquelle l'enzyme de dégradation d'oxalate est fixée à la surface par l'intermédiaire d'un agent de liaison.

22. Le dispositif médical selon une quelconque des revendications 19 à 21, dans laquelle la surface comprend en outre un composé antimicrobien.

23. Le dispositif médical selon une quelconque des revendications 19 to 22, dans laquelle l'enzyme consiste en oxalate oxidase, oxalate décarboxylase, oxalyl-CoA décarboxylase. ou formyl-CoA transférase.

24. Le dispositif médical selon une quelconque des revendications 19 to 23, dans laquelle les multiples enzymes de dégradation d'oxalate sont immobilisées sur ladite surface.
